# EUROPEAN PATENT SPECIFICATION

(11) **EP 4 253 535 B1**
(45) Date of publication and mention of the grant of the patent: **12.11.2025**
(21) Application number: 21897917.7
(22) Date of filing: 22.11.2021
(51) Int. Cl.: C08G 65/334, C08F 293/00, C09D 171/02, C12M 3/00, C12N 5/0775, C08B 37/16

(54) **POLYROTAXANE HAVING HYDROXY GROUP OR SULFO GROUP**
POLYROTAXAN MIT EINER HYDROXYLGRUPPE ODER SULFOGRUPPE
POLYROTAXANE POSSÉDANT UN GROUPE HYDROXY OU UN GROUPE SULFO

(30) Priority: 26.11.2020 JP 2020196323
(43) Date of publication of application: 04.10.2023
(73) Proprietor: Denka Company Limited, Tokyo 103-8338 (JP); Institute of Science Tokyo, Tokyo 152-8550 (JP)
(72) Inventor: AOYAMA SEKIYA, Ruriko, Tokyo 103-8338 (JP); ARISAKA, Yoshinori, Tokyo 113-8510 (JP); YUI, Nobuhiko, Tokyo 113-8510 (JP); IWATA, Takanori, Tokyo 113-8510 (JP); YODA, Tetsuya, Tokyo 113-8510 (JP); HAKARIYA, Masahiro, Tokyo 113-8510 (JP); MASUDA, Hiroki, Tokyo 113-8510 (JP)
(74) Representative: Gulde & Partner
(86) International application number: PCT/JP2021/042832
(87) International publication number: WO 2022/113944

(56) References cited:
- EP-A1- 1 419 791
- WO-A1-2017/039294
- WO-A1-2017/191827
- ARISAKA YOSHINORI, YUI NOBUHIKO: "Tethered bone morphogenetic protein-2 onto sulfonated-polyrotaxane based surfaces promotes osteogenic differentiation of MC3T3-E1 cells", JOURNAL OF BIOMATERIALS SCIENCE. POLYMER ED., vol. 28, no. 10-12, 13 August 2017 (2017-08-13), pages 974 - 985, XP009536866, DOI: 10.1080/09205063.2017.1319095
- RAJENDRAN ARUN KUMAR, ARISAKA YOSHINORI, ISEKI SACHIKO, YUI NOBUHIKO: "Sulfonated Polyrotaxane Surfaces with Basic Fibroblast Growth Factor Alter the Osteogenic Potential of Human Mesenchymal Stem Cells in Short-Term Culture", HHS AUTHOR MANUSCRIPTS, vol. 5, no. 11, 11 November 2019 (2019-11-11), US , pages 5652 - 5659, XP055869712, DOI: 10.1021/acsbiomaterials.8b01343
- ATSUSHI TAMURA, HAJIME TANAKA, NOBUHIKO YUI: "Supramolecular flower micelle formation of polyrotaxane-containing triblock copolymers prepared from macro-chain transfer agents bearing molecular hooks", POLYMER CHEMISTRY, vol. 5, no. 15, 1 January 2014 (2014-01-01), pages 4511, XP055363882, ISSN: 1759-9954, DOI: 10.1039/c4py00379a
- SEO JI-HUN, KAKINOKI SACHIRO, YAMAOKA TETSUJI, YUI NOBUHIKO: "Directing Stem Cell Differentiation by Changing the Molecular Mobility of Supramolecular Surfaces", ADVANCED HEALTHCARE MATERIALS, vol. 4, no. 2, 1 January 2015 (2015-01-01), DE , pages 215 - 222, XP055934942, ISSN: 2192-2640, DOI: 10.1002/adhm.201400173

## Description

### Technical Field

The present invention relates to the use of a polyrotaxane having a hydroxy group or a sulfo group as a surface coating for promoting osteoblast differentiation or adipocyte differentiation of mesenchymal stem cells. The invention further relates to a corresponding cell culture method and to the use of a culture substrate.

### Background Art

Cell culture technology is widely used in the pharmaceutical and cosmetic fields and regenerative medicine research, and is an important technology. As environments for cell culture, appropriate culture environments, culture solutions, and culture substrates are required. Among them, the culture substrate has a role as a scaffold to which cells adhere, and is required to have properties such as being a surface suitable for cell culture or being able to be processed, having no cytotoxicity, being able to be sterilized or maintaining a sterile state, being not deteriorated under culture conditions, and not interfering with observation with a microscope.

Conventionally, as culture substrates, glass products have been widely used, but polystyrene products are now widely used. However, a culture substrate obtained by molding a polystyrene resin is generally subjected to a surface treatment because the surface thereof is hydrophobic and affinity with cells is low. Examples of the surface treatment include a plasma treatment, a corona discharge treatment, an oxidizing agent treatment, and coating with a hydrophilic substance. As the coating agent, there are many coating agents promoting cell adhesion, and for example, Type I collagen, Type IV collagen, gelatin, fibronectin, vitronectin, laminin, matrigel, hydroxyapatite, and the like are known. It is also known that coating with water-soluble elastin promotes differentiation induction of vascular smooth muscle cells or elastin reactive cells.

Mesenchymal stem cells are somatic stem cells derived from mesoderm having self-renewal ability and pluripotency into mesenchymal tissues such as osteoblasts, chondrocytes, adipocytes, skeletal muscle cells, and ligament cells. In adult tissues, mesenchymal stem cells are present in connective tissues such as dermis, skeletal muscle, and adipose tissue and mainly in bone marrow stroma, and function to repair connective tissues, maintain homeostasis, and control proliferation and differentiation of hematopoietic stem cells. Differentiation of mesenchymal stem cells is complicatedly controlled by biological and physical factors. For example, it is known that when dexamethasone, β-glycerophosphate, or ascorbic acid is added to a medium for mesenchymal stem cells, differentiation into osteoblasts can be induced, and when dexamethasone, 3-isobutyl-1-methylxanthine, insulin, or indomethacin is added to a medium for mesenchymal stem cells, differentiation into adipocytes can be induced.

In recent years, attention has been paid to the fact that the properties (for example, bulk properties such as hardness, and surface properties of coating) of a culture substrate affect the function of cultured cells, and a study for proliferating cells having a desired function has been reported (Non-Patent Literatures 1 to 3).
Non-Patent Literature 4 discloses the use of a hydroxylated polyrotaxane as self-assembled polymeric micelles as carrier for anticancer drugs. Non-Patent Literature 5 discloses the use of a sulfonated polyrotaxane tethered with the bone morphogenetic protein-2 for promoting osteoblast differentaiation of pre-osteoblasts MC3T3-E1.
Non-Patent Literature 6 discloses osteoblast differentation comprising a step of culturing mesenchymal stem cells on sulfopropyl ether-modified PRX surfaces in the presence or absence of fibroblast growth factor in a growth medium for proliferation, and a step of inducing osteoblast differentiation on a polystyrene substrate.

### Citation List

### Non-Patent Literature

Non-Patent Literature 1: Cell, 2006, 126, 677-689.
Non-Patent Literature 2: Nature Reviews Materials, 2020, 5, 686-705.
Non-Patent Literature 3: Adv. Healthcare Mater. 2015, 4, 215-222.
Non-Patent Literature 4: Tamura et al., Polym. Chem., 2014, 5, 4511-4520.
Non-Patent Literature 5: Arisake et al., J. Biomater. Sci., Polymer Ed., 2017, 28, 974-985.
Non-Patent Literature 6: Rajendran et al., ACS Biomater. Sci. Eng. 2019, 5, 5652-5659.

### Summary of Invention

### Technical Problem

However, it is still difficult to finely adjust the bulk properties (hardness or the like) of cell culture substrate in order to obtain cells having a desired function. It is considered that selecting appropriate physical factors is useful to differentiate mesenchymal stem cells into the desired cell lineage. Therefore, an object of the present invention is to provide a coating agent and a culture substrate which can promote differentiation induction of mesenchymal stem cells.

### Solution to Problem

The present invention provides a use of a polyrotaxane as a coating agent for promoting osteoblast differentiation or adipocyte differentiation of mesenchymal stem cells, a cell culture method for promoting osteoblast differentiation or adipocyte differentiation of mesenchymal stem cells, and a use of a culture substrate in a cell culture method for promoting osteoblast differentiation or adipocyte differentiation of mesenchymal stem cells as defined in the independent claims.

### Advantageous Effects of Invention

According to the present invention, it is possible to provide a coating agent capable of promoting osteoblast differentiation and/or adipocyte differentiation of mesenchymal stem cells. According to the present invention, it is also possible to provide a substrate (culture substrate) coated with the coating agent.

### Brief Description of Drawings

FIG. 1 is a view comparing ¹H-NMR spectra of Reference Example 1, Examples 1 and 2, and Comparative Examples 1 and 2.
FIG. 2 shows mass spectra obtained by TOF-SIMS measurement of Reference Example 1, Example 1, and Comparative Example 2.
FIG. 3 shows mass spectra obtained by TOF-SIMS measurement of Comparative Example 1, Example 2, and TCPS.
FIG. 4 is a graph showing fibronectin adsorption amounts of Reference Example 1, Examples 1 and 2, and Comparative Examples 1 and 2.
FIG. 5 is a graph showing cell densities of Reference Example 1, Examples 1 and 2, and Comparative Examples 1 and 2.
FIG. 6 shows micrographs (scale bar: 100 µm) obtained by photographing cells induced to osteoblast differentiation using Reference Example 1, Examples 1 and 2, and Comparative Examples 1 and 2.
FIG. 7(A) shows photographs (scale bar: 200 µm) in which cells induced to osteoblast differentiation were stained with Alizarin Red S, using Reference Example 1, Examples 1 and 2, and Comparative Examples 1 and 2, and FIG. 7(B) is a graph comparing stained areas.
FIG. 8 shows micrographs (scale bar: 100 µm) obtained by photographing cells induced to adipocyte differentiation of Reference Example 1, Examples 1 and 2, and Comparative Examples 1 and 2.
FIG. 9(A) shows photographs (scale bar: 100 µm) in which cells induced to adipocyte differentiation were stained with Oil Red O, using Reference Example 1, Examples 1 and 2, and Comparative Examples 1 and 2, and FIG. 9(B) is a graph comparing stained areas.

### Description of Embodiments

### <Polyroxatane>

The present invention, uses a coating agent for promoting osteoblast differentiation and/or adipocyte differentiation of mesenchymal stem cells, the coating agent comprising a polyrotaxane represented by Formula (1): 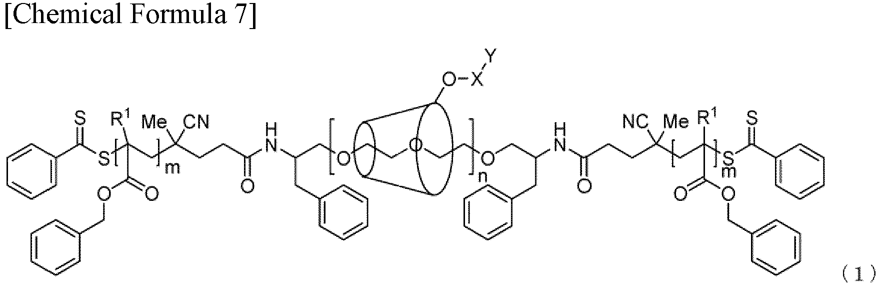 wherein R¹ is a hydrogen atom or a methyl group, m is 1 to 2000, and n is 10 to 500, 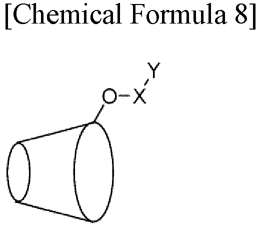 is a cyclodextrin in which at least one hydroxyl group is modified with a group represented by -X-Y, X is a divalent organic group, and Y is a hydroxyl group (-OH) or a sulfo group (-SO₃H). At least one of the cyclodextrins is contained in one molecule of the polyrotaxane.

The polyrotaxane represented by Formula (1) includes an axial molecule (linear polymer) and a modified cyclodextrin (CD, cyclic molecule), and the axial molecule threads at least one modified cyclodextrin and is capped at both ends thereof.

The axial molecule is represented by Formula (2) below, has a polyethylene glycol structure at the center of the molecule, and has a poly(meth)acrylate structure capped with a phenyldithioester group at the terminal. In the formula, R¹ is a hydrogen atom or a methyl group. The cyclodextrin is threaded to the polyethylene glycol structure portion. Each R¹ may be the same as or different from each other. R¹ is preferably a methyl group.

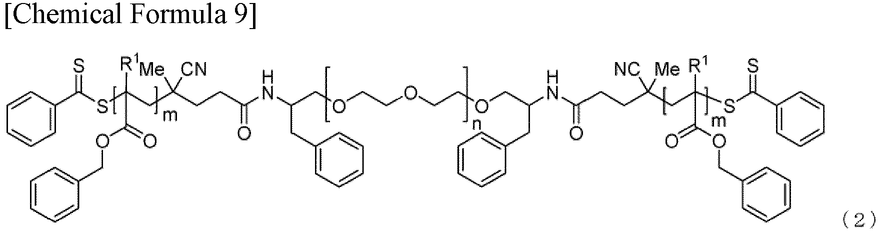

The polyethylene glycol structure may include ethylene glycol as a monomer unit and have a molecular length capable of threading at least one cyclodextrin. The number of ethylene glycol units forming the polyethylene glycol structure may be 20 to 1000, and is preferably 85 to 800 and more preferably 100 to 700. In the formula, n may be 10 to 500, and is preferably 43 to 400 and more preferably 50 to 350.

The poly(meth)acrylate structure includes benzyl (meth)acrylate as a monomer unit. In the present specification, the term "(meth)acrylate" means both "acrylate" and "methacrylate". The present inventors consider that since the poly(meth)acrylate structure has an action of enhancing adhesion to a substrate such as polystyrene, the polyethylene glycol moiety threading the cyclodextrin is separated from the substrate in a loop shape. The number m of benzyl (meth)acrylate units forming the poly(meth)acrylate structure may be 1 to 2000, and is preferably 20 to 1000 and more preferably 30 to 500, per polybenzyl (meth)acrylate at one terminal of the triblock copolymer. Each m may be the same as or different from each other.

The modified cyclodextrin used in the present embodiment is represented by Formula (3) below, and at least one hydroxyl group of glucose constituting the cyclodextrin is modified with -X-Y. The cyclodextrin may be any of α-cyclodextrin, β-cyclodextrin, γ-cyclodextrin, and combinations thereof. A preferred cyclodextrin is α-cyclodextrin. The following is a figure schematically showing a cyclodextrin modified with -X-Y. "-O-X-Y" in the figure indicates that a hydroxyl group in glucose constituting the cyclodextrin is modified with -X-Y. In the figure, only one "-O-X-Y" is shown, but it should not be interpreted restrictively that only one hydroxyl group is modified with -X-Y.

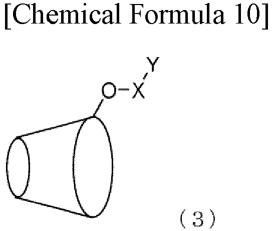

In the polyrotaxane, at least one modified cyclodextrin is threaded by an axial molecule. The number of cyclodextrins per polyrotaxane molecule can be independently determined and need not be uniquely determined by the molecular length of the polyethylene glycol structure. Stoichiometrically, since the cyclodextrin can include two units of ethylene glycol, which is a repeating unit of polyethylene glycol, the number of cyclic molecules has an upper limit depending on the molecular weight of the linear polymer to be used. For example, the number of cyclodextrins per polyethylene glycol molecule having a number average molecular weight of 20000 may be 5 to 150, preferably 5 to 120, and more preferably 5 to 100. However, the length (total length when the polyrotaxane has a plurality of cyclodextrins) of the axial molecule of the cyclodextrin in the main chain direction does not exceed the molecular length of polyethylene glycol.

The number of hydroxyl groups modified with -X-Y in the cyclodextrin may be 1 to 18, and is preferably 1 to 10 and more preferably 2 to 8, with respect to one cyclodextrin. When the number of hydroxyl groups modified with -X-Y is within the above range, the effect of promoting adipocyte differentiation and/or osteoblast differentiation of mesenchymal stem cells is more excellent. When the number of hydroxyl groups modified with -X-Y is 10 or less, solubility in an organic solvent such as dimethyl sulfoxide (DMSO) is more excellent. When the number of hydroxyl groups modified with -X-Y is 2 or more, the amount of change in molecular mobility, the chemical composition of the surface, and the amount of change in physicochemical properties (for example, contact angle and zeta potential) of the surface increase. Since the molecular mobility, the chemical composition of the surface, and the physicochemical properties (for example, contact angle and zeta potential) of the surface of the polyrotaxane can be adjusted by the number of hydroxyl groups modified with -X-Y, a polyrotaxane surface having molecular mobility suitable for promotion of adipocyte differentiation and/or osteoblast differentiation of mesenchymal stem cells can be produced.

X is a divalent organic group, and is not particularly limited. Examples of the organic group include an alkylene group having 1 to 10 carbon atoms, an alkenylene group having 1 to 20 carbon atoms, and an alkynylene group having 1 to 20 carbon atoms, and the organic group may have an oxo group at any position, may be through an oxy group or an imino group, and may be a combination thereof. For example, a combination of a carbon atom having an oxo group and an oxy group forms an ester bond, and a combination of a carbon atom having an oxo group and an imino group forms an amide bond. The alkylene group having a plurality of oxy groups is also referred to as an oxyalkylene group, and includes, for example, a polyoxyethylene group having 1 to 10 carbon atoms and a polyoxypropylene group having 1 to 10 carbon atoms. Specific examples of the organic group include alkylene groups such as a methylene group, an ethylene group, a propylene group, a butylene group, a pentylene group, a hexylene group, a heptylene group, an octylene group, a nonanylene group, and a decylene group; alkenylene groups such as a propynylene group, a butenylene group, a pentenylene group, a hexenylene group, a heptenylene group, an octenylene group, a nonenylene group, and a decenylene group; and alkynylene groups such as a propargyl group, a butynylene group, a pentynylene group, a hexynylene group, a heptynylene group, an octynylene group, a nonylene group, and a decynylene group. An organic group having an oxo group, an oxy group, or an imino group preferably forms an ester bond, a carbonate bond, or a urethane bond together with an oxygen atom derived from the hydroxyl group of the cyclodextrin. Examples of the organic group having an oxo group, an oxy group, or an imino group include carbonyl alkylene groups such as a carbonyl methylene group (-C(=O)CH₂-); alkylene carbonyl groups such as a methylenecarbonyl group (-CH₂C(=O)-); carbonylaminoalkylene groups such as a carbonylaminomethylene group (-C(=O)NHCH₂-); alkylenecarbonylamino groups such as a methylenecarbonylamino group (-CH₂C(=O)NH-); carbonylaminoalkylene groups such as a carbonylaminoethylene group (-C(=O)NHCH₂CH₂-), carbonylaminoalkylene oxyalkylene groups such as a carbonylaminoethyleneoxyethylene group (-C(=O)NHCH₂CH₂OCH₂CH₂-); and oxyalkylene groups such as an oxypropylene group (-OCH₂CH₂CH₂-).

The modified cyclodextrin is preferably represented by Formula (4). In the formula, -C(=O)NH-Xa- corresponds to X in Formula (3), and corresponds to one of aspects in which X has an oxo group and an imino group. Xa is an organic group having 1 to 9 carbon atoms, preferably an alkylene group having 2 to 9 carbon atoms, and more preferably a polyoxyethylene group having 2 to 8 carbon atoms.

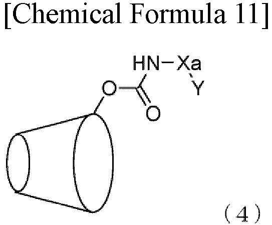

The polyrotaxane includes a structure in which an axial molecule threads at least one cyclodextrin. Each cyclodextrin can move along the main chain direction of the axial molecule and rotate about the main chain of the axial molecule. Such structural properties are referred to as molecular mobility. The molecular mobility can vary, for example, according to the number of cyclodextrins, the number of substituents modified to glucose constituting the constituting cyclodextrin.

The molecular mobility can be evaluated by coating the surface of a culture substrate, then measuring the static contact angle of the coating surface using a contact angle meter, and using a droplet method and a captive bubble method. For the evaluation of molecular mobility, for example, methods described in Soft Matter, 2012, 8, 5477-5485 (Ji-Hun Seo et al.), Adv. Healthcare Mater. 2015, 4, 215-222, and the like may be referred to. Specifically, the contact angle hysteresis value of the coating surface can be calculated from a difference between the contact angle of water (contact angle of water in the atmosphere) measured by the droplet method and the contact angle of water (contact angle of water in water) determined from the contact angle of air bubbles measured by the captive bubble method. As a control, comparison with the effect on a culture substrate coated with DMSO may be conducted.

The molecular mobility can be adjusted by changing the number of threaded cyclodextrins and/or the number of hydroxyl groups modified with -X-Y in the cyclodextrin. For example, when the number of threaded cyclodextrins is 3 to 120, it can be more suitable for promoting adipocyte differentiation and/or osteoblast differentiation of mesenchymal stem cells. When the number of threaded cyclodextrins is large, the cyclodextrin is easily purified by reprecipitation.

The content of the polyrotaxane may be 0.0005 to 5 mass%, preferably 0.01 to 1 mass%, and more preferably 0.02 to 0.5 mass%, based on the mass of the coating agent.

The coating agent of the present embodiment may comprise a solvent and an optional additive in addition to the above-described polyrotaxane. Examples of the solvent include dimethyl sulfoxide (DMSO), tetrahydrofuran (THF), N,N-dimethylformamide (DMF), methanol, 2-propanol, chloroform, and methylene chloride. Examples of such an additive include an antioxidant.

The coating agent of the present embodiment is used for coating a surface of a substrate that can be used as a culture substrate. The substrate that can be used as a culture substrate may be a substrate well known to those skilled in the art. Examples of the material for the substrate include glass, polystyrene, polypropylene, polyethylene, polyolefin, polycarbonate, and an acrylic block copolymer (BCF).

According to the coating agent of the present embodiment, by adjusting the number of hydroxyl groups modified with -X-Y in glucose constituting the cyclodextrin, it is possible to adjust promotion of adipocyte differentiation and/or osteoblast differentiation of mesenchymal stem cells without depending on the bulk properties of the substrate itself. More specifically, as adipocyte differentiation progresses, the amount of lipid droplets accumulated in cells increases. Lipid droplets accumulated in cells can be stained with Oil Red O dye to evaluate the degree of lipid droplet accumulation in cells. Adipocyte differentiation of mesenchymal stem cells may be determined by the expression level of a differentiation marker gene (for example, PPARγ, C/EBPα, and aP2), which is an index indicating adipocyte differentiation. In osteoblast differentiation, as the differentiation progresses, bone nodules are formed (also called calcification). The bone nodules can be stained with Alizarin Red S to evaluate the extent of calcification. Osteoblast differentiation of mesenchymal stem cells may be determined by the expression level of a differentiation marker gene (for example, RUNX2, alkaline phosphatase, osteocalcin, osteopontin, bone sialoprotein, and Type I collagen), which is an index indicating osteoblast differentiation.

The polyrotaxane can be produced with reference to Examples, and may be produced as follows. Hydroxyl groups at both ends of polyethylene glycol having a desired length are converted into leaving groups (for example, halogenation, methanesulfonylation, or toluenesulfonylation), and etherified with phenylalaninol to obtain a diamine. The obtained diamine is mixed with a cyclodextrin to obtain a pseudo-rotaxane. At this time, the number of threaded cyclodextrins can be adjusted by adjusting the amount of cyclodextrin per diamine molecule. Subsequently, the mixture is reacted with CPADB and DMT-MM to cap the cyclodextrin so as not to be removed from the axial molecule, and then benzyl methacrylate (corresponding to a (meth)acrylate structure) is introduced at both terminals as an anchoring segment by a reversible addition-fragmentation chain transfer polymerization reaction (RAFT polymerization reaction).

### <Cell Culture Method for Promoting Adipocyte Differentiation>

The present invention provides a cell culture method for promoting adipocyte differentiation of mesenchymal stem cells, the method comprising: culturing mesenchymal stem cells on a surface of a substrate coated with a composition comprising a polyrotaxane represented by Formula (1), wherein for promoting osteoblast differentiation, the mesenchymal stem cells are cultured on the surface of the substrate in a medium for osteoblast differentiation, and for promoting adipocyte differentiation, the mesenchymal stem cells are cultured on the surface of the substrate in a medium for adipocyte differentiation.

In the present invention, as the "polyrotaxane represented by Formula (1)", those described above is referred to, and as the "composition comprising a polyrotaxane represented by Formula (1)", the coating agent described above is used.

In the cell culture method of the present embodiment, as the culture substrate, a substrate coated with a composition comprising a polyrotaxane represented by Formula (1) is used. Cells are cultured by adhering the cells to the surface of the coated substrate.

The culture substrate is obtained by applying a coating comprising a polyrotaxane represented by Formula (1) to a substrate. The substrate may be a substrate well known to those skilled in the art. Examples of the material for the substrate include glass, polystyrene, polypropylene, polyethylene, polyolefin, polycarbonate, and an acrylic block copolymer (BCF). The substrate may be a commercially available glass substrate or plastic substrate.

In the cell culture method of the present disclosure, a medium is put on the coating surface provided on the substrate so that the cells are immersed, and the cells to be cultured are seeded and cultured. The medium may be replaced with a new medium as necessary. A step of injecting a medium for proliferation before differentiating the cells to proliferate the seeded cells may be provided. In this case, after the cells are proliferated until a sufficient number of cells is obtained, the medium for proliferation is replaced with a medium for differentiation. As the medium for proliferation and the medium for differentiation, a medium well known to those skilled in the art can be used.

As a medium for adipocyte differentiation of mesenchymal stem cells, for example, Mesenchymal Stem Cell Adipogenic Differentiation Medium 2 (C-28016) available from PromoCell GmbH (Heidelberg, Germany) can be used.

The cell culture environment can be arbitrarily set under the conditions well known to those skilled in the art.

Adipocyte differentiation of mesenchymal stem cells means differentiation of mesenchymal stem cells into adipocytes. As adipocyte differentiation progresses, the amount of lipid droplets accumulated in cells increases. The effect of promoting adipocyte differentiation of mesenchymal stem cells can be evaluated by cell staining with Oil Red O (staining of lipid droplets in cells). The effect may be determined by measuring a change in the expression level of a differentiation marker. Examples of the differentiation marker of adipocyte differentiation include PPARγ, C/EBPα, and aP2. When differentiation is statistically significantly promoted as compared with the case of using a culture substrate in which a substrate (made of glass or polystyrene) is coated with an unmodified polyrotaxane (for example, PRX-PBzMA described below), it can be determined that the effect of promoting adipocyte differentiation of mesenchymal stem cells is exhibited.

The cell culture method of the present disclosure may comprise coating the composition comprising a polyrotaxane represented by Formula (1) onto the surface of the substrate.

The composition comprising a polyrotaxane represented by Formula (1) can be coated on a substrate. The coating method is not particularly limited, and examples thereof include casting, spin coating, gravure coating, die coating, knife coating, bar coating, blade coating, and roll coating. A preferred coating method is casting.

### <Cell Culture Method for Promoting Osteoblast Differentiation>

The present invention further provides a cell culture method for promoting osteoblast differentiation of mesenchymal stem cells, the method comprising: culturing mesenchymal stem cells on a surface of a substrate coated with a composition comprising a polyrotaxane represented by Formula (1).

In the present invention, as the "polyrotaxane represented by Formula (1)", those described above are referred to, and as the "composition comprising a polyrotaxane represented by Formula (1)", the coating agent described above is used.

In the cell culture method of the present invention, as the culture substrate, a substrate coated with a composition comprising a polyrotaxane represented by Formula (1) is used. Cells are cultured by adhering the cells to the surface of the coated substrate.

The culture substrate is obtained by applying a coating comprising a polyrotaxane represented by Formula (1) to a substrate. The substrate may be a substrate well known to those skilled in the art. Examples of the material for the substrate include glass, polystyrene, polypropylene, polyethylene, polyolefin, polycarbonate, and an acrylic block copolymer (BCF). The substrate may be a commercially available glass substrate or plastic substrate.

In the cell culture method of the present disclosure, a medium is put on the coating surface provided on the substrate so that the cells are immersed, and the cells to be cultured are seeded and cultured. The medium may be replaced with a new medium as necessary. A step of injecting a medium for proliferation before differentiating the cells to proliferate the seeded cells may be provided. In this case, after the cells are proliferated until a sufficient number of cells is obtained, the medium for proliferation is replaced with a medium for differentiation. As the medium for proliferation and the medium for differentiation, a medium well known to those skilled in the art can be used.

As a medium for osteoblast differentiation of mesenchymal stem cells, for example, Mesenchymal Stem Cell Osteogenic Differentiation Medium (C-28013) available from PromoCell GmbH (Heidelberg, Germany) can be used.

The cell culture environment can be arbitrarily set under the conditions well known to those skilled in the art.

Osteoblast differentiation of mesenchymal stem cells means differentiation of mesenchymal stem cells into osteoblasts. As osteoblast differentiation progresses, bone nodules are formed (also called calcification). The effect of promoting osteoblast differentiation of mesenchymal stem cells can be evaluated by cell staining with Alizarin Red S (bone nodules are stained). The effect may be determined by measuring a change in the expression level of a differentiation marker. Examples of the differentiation marker of osteoblast differentiation include RUNX2, alkaline phosphatase, osteocalcin, osteopontin, bone sialoprotein, and Type I collagen. When differentiation is statistically significantly promoted as compared with the case of using a culture substrate in which a substrate (made of glass or polystyrene) is coated with an unmodified polyrotaxane (for example, PRX-PBzMA described below), it can be determined that the effect of promoting osteoblast differentiation of mesenchymal stem cells is exhibited.

The cell culture method of the present disclosure may comprise coating the composition comprising a polyrotaxane represented by Formula (1) onto the surface of the substrate.

The composition comprising a polyrotaxane represented by Formula (1) can be coated on a substrate. The coating method is not particularly limited, and examples thereof include casting, spin coating, gravure coating, die coating, knife coating, bar coating, blade coating, and roll coating. A preferred coating method is casting.

### <Use of Culture Substrate >

The present invention further provides a use of a culture substrate comprising a substrate coated with a composition comprising a polyrotaxane represented by Formula (1).

In the present invention, as the "polyrotaxane represented by Formula (1)", those described above are referred to, and as the "composition comprising a polyrotaxane represented by Formula (1)", the coating agent described above is used.

The coated substrate (culture substrate) of the present disclosure is particularly suitable as a culture substrate for promoting adipocyte differentiation and/or osteoblast differentiation of mesenchymal stem cells.

According to the culture substrate of the present disclosure, by adjusting the number of hydroxyl groups modified with -X-Y in glucose constituting the cyclodextrin, it is possible to adjust differentiation (promotion of adipocyte differentiation and/or osteoblast differentiation) of mesenchymal stem cells and proliferation (promotion or suppression) of mesenchymal stem cells without depending on the bulk properties of the substrate itself.

### Examples

Hereinafter, the present invention will be described in more detail with reference to Examples. However, the present invention is not limited thereto. The abbreviations used in the examples are conventional abbreviations well known to those skilled in the art, and the meanings of some abbreviations are shown below.
αCD: α-cyclodextrin
CDI: carbonyl diimidazole
CPADB: 4-cyanopentanoic acid dithiobenzoate
DMEM: Dulbecco's modified Eagle medium
DMF: N,N-dimethylformamide
DMSO: dimethyl sulfoxide
DMT-MM: 4-(4,6-dimethoxy-1,3,5-triazine-2-yl)-4-methylmorpholinium chloride
EDTA: ethylenediaminetetraacetic acid
FBS: fetal bovine serum
IBMX: 3-isobutyl-1-methylxanthine
α-MEM: α-minimum essential medium
MeOH: methanol
MsCl: methanesulfonic acid chloride
PBS: phosphate buffered saline
PEG: polyethylene glycol
TCPS: polystyrene for cell culture
TEA: triethylamine
THF: tetrahydrofuran
¹H-NMR: proton nuclear magnetic resonance spectrometry

### 1. Synthesis of Polyrotaxane

### (Example 1)

A method for synthesizing a polyrotaxane is shown below.

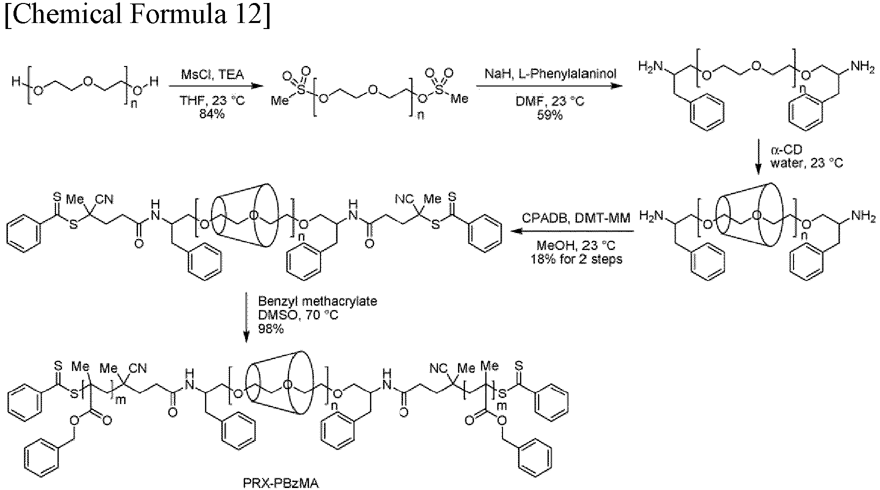

### Step 1: Synthesis of α,ω-Bismesyl polyethylene glycol

Polyethylene glycol having a number average molecular weight of 20000 (25.0 g, 1.25 mmol) and TEA (5.3 mL, 37.5 mmol) were dissolved in anhydrous THF (130 mL), MsCl (2.0 mL, 25.0 mmol) was added dropwise, and the mixture was stirred at 23°C. After 5 hours, the reaction solution was filtered, the filtrate was precipitated with diethyl ether, and the precipitate was collected as a solid. The obtained precipitate was dried under reduced pressure to obtain α,ω-bismesyl polyethylene glycol (21.1 g, yield: 84%).

### Step 2: Synthesis of Bis(2-amino-3-phenylpropyl)polyethylene glycol

L-Phenylalaninol (1.49 g, 9.85 mmol) and sodium hydride (0.971 g, 60% mineral oil) were dissolved in anhydrous DMF (86 mL) under a nitrogen atmosphere. To this mixed liquid, α,ω-bismesyl polyethylene glycol (20.0 g, 0.992 mmol) was added, and the mixture was stirred at 23°C. After 24 hours, the reaction solution was filtered, the filtrate was precipitated with diethyl ether, and the precipitate was collected as a solid. The obtained precipitate was dried under reduced pressure to obtain bis(2-amino-3-phenylpropyl)polyethylene glycol (11.8 g, yield: 59%).

### Step 3: Synthesis of Pseudo-polyrotaxane

Bis(2-amino-3-phenylpropyl)polyethylene glycol (10.1 g, 0.499 mmol) was dissolved in water (50 mL), a saturated aqueous solution (380 mL) of αCD (55.2 g, 56.6 mmol) was added thereto, and the mixture was stirred at 23°C. After 19 hours, the reaction solution was centrifuged to collect a precipitate, and freeze-dried for 9 days to obtain a pseudo-polyrotaxane as a crude product.

### Step 4: Synthesis of Polyrotaxane PRX-CPADB

CPADB (5.50 g, 19.7 mmol) and DMT-MM (5.50 g, 19.9 mmol) were dissolved in methanol (500 mL), and the pseudo-polyrotaxane obtained above was added to the reaction solution at 23°C, followed by stirring. After 1 day, the crude product was washed with methanol, reprecipitated with hydrous DMSO, centrifuged and lyophilized for 9 days to obtain the polyrotaxane PRX-CPADB (11.9 g, 2-step yield: 18%) as a powder. The structure of polyrotaxane PRX-CPADB was confirmed by ¹H-NMR (solvent: DMSO-d₆). The number of threaded αCD was determined by ¹H-NMR (solvent: D₂O).

¹H-NMR(500 MHz, DMSO-d₆) δ 3.12-3.91 (m, PEG backbone and H2, H3, H4, H5,and H6 protons of αCD), 4.43 (m, OH6 of αCD), 4.80 (m, H1 of αCD), 5.49 (m, OH3 of αCD), 5.65 (m, OH2 of αCD), 7.17 (t, aromatics of phenylalanyl group), 7.25(t, aromatics of phenylalanyl group), 7.52 (t, aromatics of CPADB group), 7.70(t, aromatics of CPADB), and 7.91 (t, aromatics of CPADB).

### Step 5: Synthesis of Polyrotaxane (PRX-PBzMA) of Reference Example 1

Polyrotaxane PRX-CPADB (1.50 g, 13.9 µmol) was dissolved in anhydrous DMSO (12 mL), and benzyl methacrylate (1.71 g, 9.70 mmol) and 4,4'-azobis(4-cyanovaleric acid) (1.55 mg, 5.54 µmol) were added to this mixed liquid. After degassing by FPT cycle (Freeze-Pump-Thaw cycle), the mixture was stirred at 70°C. After 1 day, the crude product was precipitated with diethyl ether and dried under reduced pressure to obtain the polyrotaxane PRX-PBzMA (3.13 g, yield: 98%). The number m of benzyl methacrylate units forming the polybenzyl methacrylate structure was determined by ¹H-NMR (solvent: DMSO-d₆).

¹H-NMR(500 MHz, DMSO-d₆) δ 0.40-0.95 (m, -CH(-CH3)-CH2-of PBzMA),1.43-2,10 (m, -CH(-CH3)- CH2- of PBzMA), 3.17-4.02 (m, PEG backbone and H2, H3, H4, H5, and H6 protons of αCD), 4.44 (m, OH6 of αCD), 4.80 (m, H1 of αCD), 4.86 (m, -CH2-Ph of PBzMA), 5.49 (m, OH3 of αCD), 5.66 (m, OH2 of αCD), and 7.26 (m, aromatics of PBzMA).

### Step 6a: Synthesis of Polyrotaxane (NH₂-PRX) of Comparative Example 1

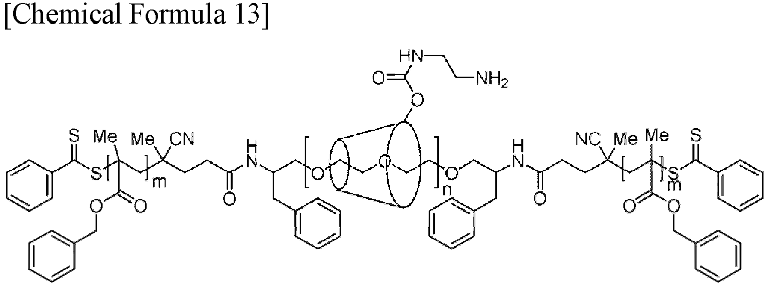

Polyrotaxane PRX-PBzMA (200 mg, 0.895 µmol) was dissolved in anhydrous DMSO (10 mL) and then CDI (104 mg, 0.643 mmol) was added to the solution. After stirring at 23°C for 1 day, ethylenediamine (0.43 mL, 6.43 mmol) was added to the solution, and the mixture was further stirred at 23°C for 1 day. Next, the reaction solution was purified by dialysis for 4 days. The product was freeze-dried for 7 days to obtain NH₂-PRX (176 mg, yield: 73%) as a powder. The number of amino groups was determined by ¹H-NMR analysis (solvent: DMSO-d₆).

¹H-NMR (500 MHz, DMSO-d₆) δ = 0.38-0.92 (m, -CH(-CH3)-CH2- of PBzMA),1.47-2.02 (m, -CH(-CH3)- CH2- of PBzMA), 3.00 (m, -O-CO-NH-CH2-CH2-NH2), 3.15-4.55 (m, PEG backbone, H2, H3, H4, H5, and H6 protons of α-CD, and OH6 of α-CD), 4.86 (m, -CH2-Ph of PBzMA, H1 of α-CD), and 7.26 (m, aromatics of PBzMA).

### Step 6b: Synthesis of Polyrotaxane (CH₃-PRX) of Comparative Example 2

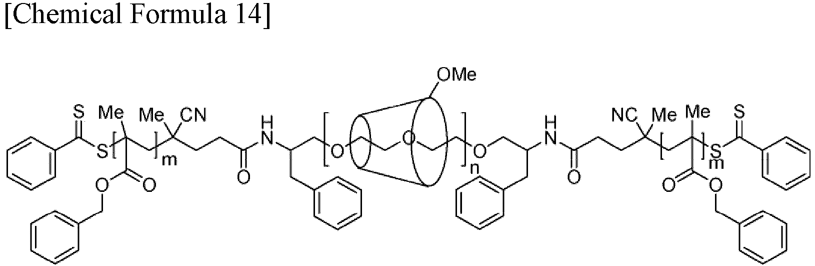

Polyrotaxane PRX-PBzMA (0.15 g, 0.671 µmol) was dissolved in anhydrous DMSO (1.31 mL), sodium hydroxide (28.7 mg, 0.716 mmol) and iodomethane (15 µL, 0.239 mmol) were added thereto, and the mixture was stirred at 23°C. After 1 hour, methanol was added to stop the reaction, and the mixture was dialyzed for 3 days to obtain a polyrotaxane (115 mg, yield: 75%) of Comparative Example 1. The number of methyl groups was determined by ¹H-NMR analysis (solvent: DMSO-d₆).

¹H-NMR (500 MHz, DMSO-d₆) δ 0.40-0.95(m, -CH(-CH3)-CH2-of PBzMA), 1.43-2.10 (m, -CH(-CH3)- CH2- of PBzMA),3.17-4.02 (m, PEG backbone, H2, H3, H4, H5, and H6 protons of αCD, and -OCH3 of αCD), 4.44 (m, OH6 of αCD), 4.57-5.10 (m, H1 of αCD and -CH2-Ph of PBzMA), 5.49 (m, OH3 of αCD), 5.64 (m, OH2 of αCD), and 7.26 (m, aromatics of PBzMA).

### Step 6c: Synthesis of Polyrotaxane (OH-PRX) of Example 1

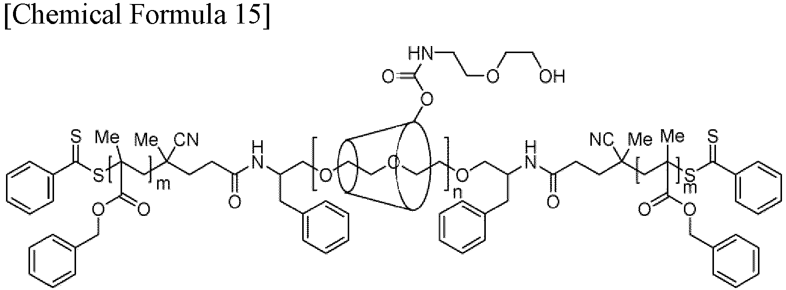

Polyrotaxane PRX-PBzMA (200 mg, 0.895 µmol) was dissolved in anhydrous DMSO (10 mL) and CDI (130 mg, 0.804 mmol) was added to the solution. After stirring at 23°C for 1 day, 2-(2-aminoethoxy)ethanol (0.80 mL, 8.04 mmol) was added to the solution, and the mixture was further stirred at 23°C for 1 day. Next, the reaction solution was purified by dialysis for 4 days. The product was freeze-dried for 7 days to obtain OH-PRX (194 mg, yield: 74%) as a powder. The number of OH groups was determined by ¹H-NMR analysis (solvent: DMSO-d₆).

1H-NMR (500 MHz, DMSO-d₆) δ 0.42-0.95 (m, -CH(-CH3)-CH2- of PBzMA), 1.45-2.02(m, -CH(-CH3)- CH2- of PBzMA), 3.14 (m, -O-CO-NH-CH2-CH2-O-CH2-CH2-OH),3.17-4.70 (m, PEG backbone, H2, H3, H4, H5, and H6 protons of α-CD, OH6 of α-CD, and -O-CO-NH-CH2-CH2-O-CH2-CH2-OH), 4.86 (m, -CH2-Ph of PBzMA, H1 of α-CD), and 7.26 (m, aromatics of PBzMAH).

### Step 6d: Synthesis of Polyrotaxane (SO₃H-PRX) of Example 2

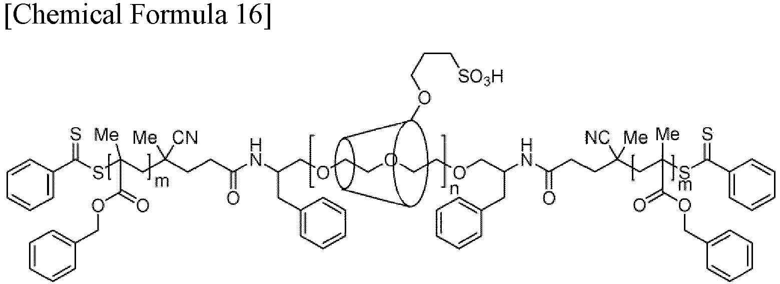

Polyrotaxane PRX-PBzMA (150 mg, 0.671 µmol) was dissolved in anhydrous DMSO (3.0 mL) and then NaOH powder (65.1 mg, 1.63 mmol) and 1,3-propane sultone (66.3 mg, 0.543 mmol) were added to the solution. After stirring at 23°C for 1 day, the mixture was dialyzed and purified for 5 days. The product was freeze-dried for 7 days to obtain SO₃H-PRX (155 mg, yield: 68%) as a powder. The number of sulfo groups (-SO₃H) was determined by ¹H-NMR analysis (solvent: DMSO-d₆).

1H-NMR (500 MHz, DMSO-d₆) δ = 0.33-0.95 (m, -CH(-CH3)-CH2- of PBzMA),1.41-2.12 (m, -CH(-CH3)- CH2- of PBzMA and -O-CH2-CH2-CH2-SO3Na),2.92-4.23 (m, PEG backbone, H2, H3, H4, H5, and H6 protons of α-CD, and -O-CH2-CH2-CH2-SO3Na),4.86 (m, -CH2-Ph of PBzMA, H1 of α-CD), 7.26 (m, aromatics of PBzMA).

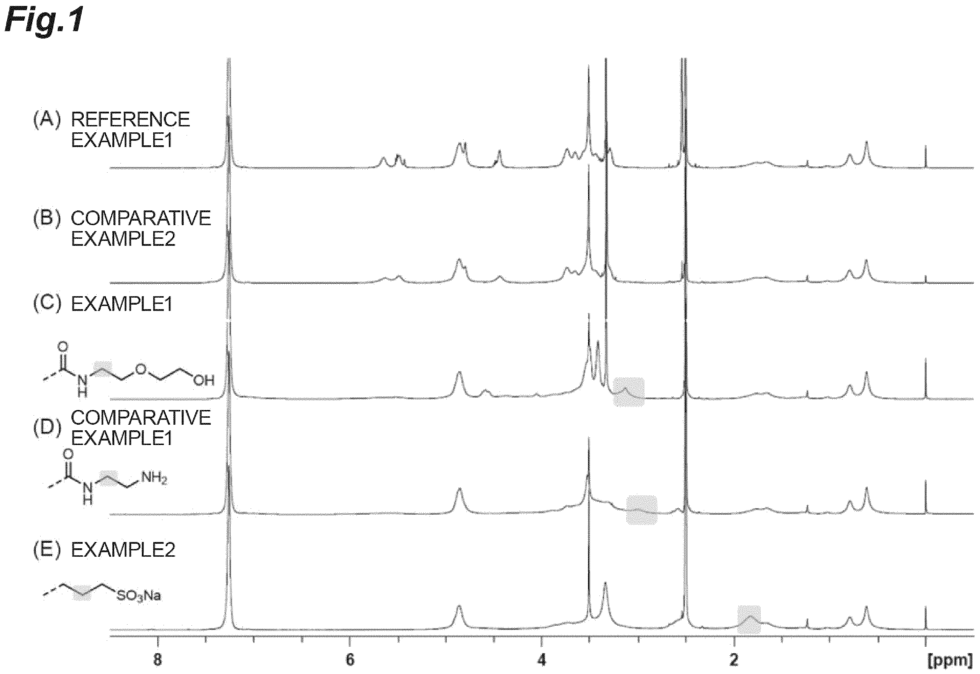

Data of the obtained polyrotaxane are shown in Table 1. FIG. 1 is a view comparing ¹H-NMR spectra of polyrotaxanes of Reference Example 1, Examples 1 and 2, and Comparative Examples 1 and 2. Characteristic peaks in each spectrum were shown in gray. The number in parentheses indicates the number of functional group modifications per cyclodextrin.

**[Table 1]**

| | Number average molecular weight of PEG moiety | Number average molecular weight of polybenzyl methacrylate | Number of threaded αCD | Number of functionalized hydroxyl groups |
|---|---|---|---|---|
| Reference Example 1 | 20000 | 115200 | 89.8 | 0 (0) |
| Comparative Example 1 | 20000 | 115200 | 89.8 | 310 (3.5) |
| Comparative Example 2 | 20000 | 115200 | 89.8 | 312 (3.5) |
| Example 1 | 20000 | 115200 | 89.8 | 392 (4.4) |
| Example 2 | 20000 | 115200 | 89.8 | 568 (6.3) |

The obtained polyrotaxane was dissolved in DMSO to produce a coating agent. The obtained coating agent was cast on TCPS.

### 2. Analysis of Surface Chemical Composition

### (1) Elemental Composition of Surface

The chemical composition of the polyrotaxane surface was analyzed by TOF-SIMS(PHI NanoTOF II, ULVAC-PHI) with 30 keV Bi3⁺⁺ primary ions. The analysis field is 100×100 µm. The elemental composition of the surface was determined by X-ray photoelectron spectroscopy (Al-Kα, Thermo Fisher Scientific, East Grinstead) using monochromatized X-ray radiation with an energy of 1486.6 eV. The diameter of the analysis region was 400 µm, and the elemental composition was calculated from the average of three points on each surface.

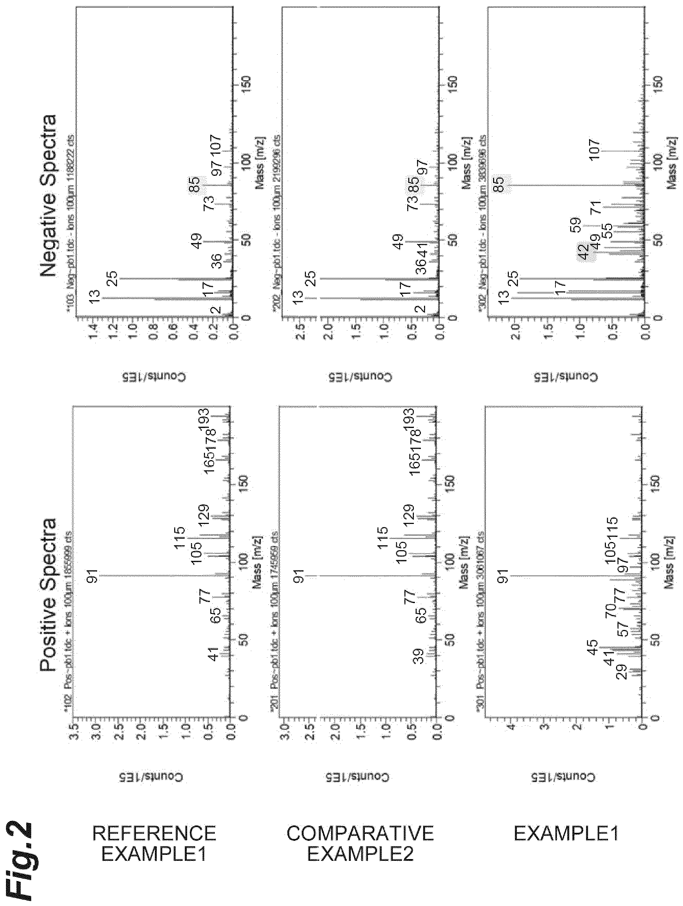

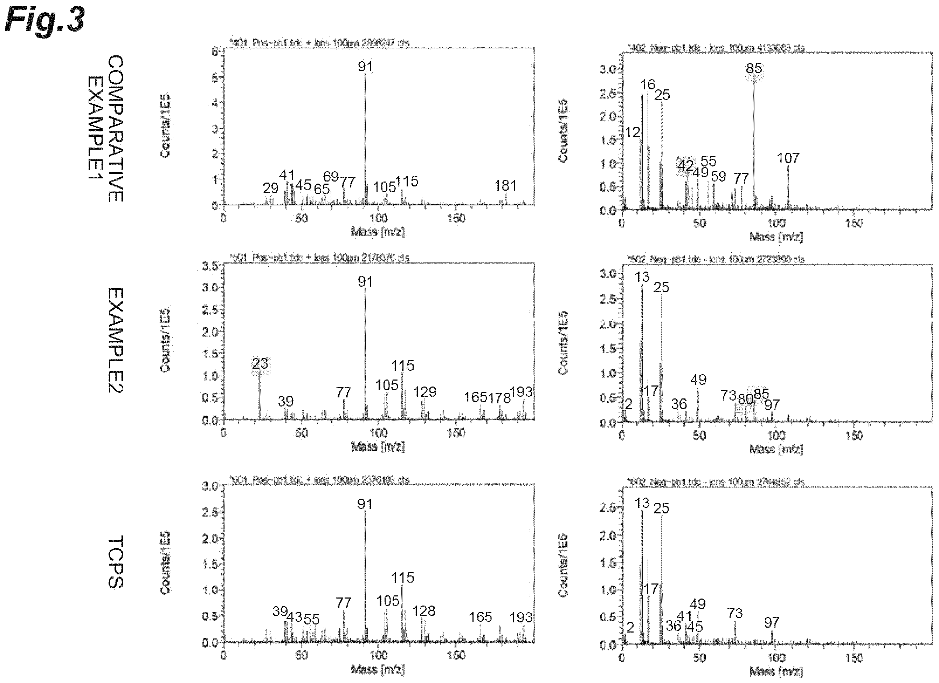

FIGS. 2 and 3 show mass spectra obtained by TOF-SIMS measurement. A characteristic peak at m/z = 85 was observed on the surface other than TCPS. This peak suggested the presence of a methacrylate group (C₄H₅O₂⁻), and it was speculated that the TCPS surface was coated with a triblock copolymer having a PBzMA structure. Characteristic peaks at m/z = 42 suggesting the presence of an N-containing group (CNO⁻) were detected on the surfaces of Comparative Example 1 and Example 1. From the results of the XPS analysis shown in Table 2, the elemental composition of nitrogen and oxygen was significantly higher on the NH₂-PRX surface (Comparative Example 1) and OH-PRX (Example 1) than on other surfaces.

**[Table 2]**

| | Element (atom%) | | | |
|---|---|---|---|---|
| | C1s | O1s | N1s | S2p |
| Reference Example 1 | 95.8±0.3 | 4.0±0.5 | 0.2±0.1 | 0.0±0.0 |
| Comparative Example 1 | 78.8±0.2 | 19.1±0.3 | 2.1±0.1 | 0.0±0.0 |
| Comparative Example 2 | 94.0±2.4 | 5.8±2.3 | 0.3±0.1 | 0.0±0.0 |
| Example 1 | 77.7±0.4 | 20.4±0.4 | 1.9±0.0 | 0.0±0.0 |
| Example 2 | 92.6±2.7 | 6.5±2.3 | 0.2±0.2 | 0.3±0.2 |

### (2) Measurement of Contact Angle and Zeta Potential

The coatings comprising polyrotaxane of Examples 1 and 2 and Comparative Examples 1and 2 were applied to the surface of polystyrene for cell culture (TCPS). The static contact angle of the coating surface was measured using a contact angle meter (trade name: DM-501, manufactured by Kyowa Interface Science Co., Ltd. by both of a droplet method and a captive bubble method. The contact angle hysteresis value of each surface was calculated from a difference between the contact angle of water (contact angle of water in the atmosphere) measured by the droplet method and the contact angle of water (contact angle of water in water) determined from the contact angle of air bubbles measured by the captive bubble method. All measurement values were obtained on four different surfaces, and an average value of three different points on each surface was recorded. It is known that a difference in contact angle hysteresis indicates a difference in molecular mobility between surfaces.

The contact angle and the contact angle hysteresis of the polyrotaxane surfaces of Reference Example 1, Examples 1 and 2, and Comparative Examples 1 and 2 are shown in Table 3. The contact angle of water in the atmosphere on the polyrotaxane surface was in a range of 80 to 100°, and it was confirmed that wettability with the TCPS surface (contact angle: 74°) surface was changed by the polyrotaxane coating. The contact angles of water in the atmosphere on the polyrotaxane surfaces of Examples 1 and 2 and Comparative Examples 1 and 2 were slightly smaller than the contact angle of the polyrotaxane of Reference Example 1, and there was no significant difference between the polyrotaxane surfaces. On the other hand, the contact angle of water in water measured using the captive bubble method in water tended to be different, and a significant difference was observed between the polyrotaxane surfaces.

The polyrotaxane surface has molecular mobility derived from the interlocking structure between αCD and the polyethylene glycol chain and exhibits unique behavior in the hydrated state. For example, it is known that the molecular mobility upon hydration of the polyrotaxane surface is related to (1) a result obtained from dissipation energy loss (QCM-D) measured in water using a quartz crystal microbalance with dissipation and (2) a result of the contact angle hysteresis measured by a difference between a contact angle of water in air and a contact angle of water obtained from a contact angle of bubbles in water. Since the value of the contact angle hysteresis changed depending on the type of surface functional group on each coating surface using Examples 1 and 2 and Comparative Examples 1 and 2, functional group modification of the hydroxyl group of the cyclodextrin is considered to be useful for adjusting molecular mobility.

The zeta potential of the polyrotaxane surface was measured using quartz flow cell for a flat plate sample using an electrophoretic light scattering spectrometer (ELSZ-2, Otsuka Electronics Co., Ltd.). The mobility of electroosmosis on the surface was analyzed using monitoring particles (Otsuka Electronics Co., Ltd.) in 10 mM PBS to calculate the zeta potential on the surface. All measurements were performed on three different surfaces.

The zeta potentials of the polyrotaxane surfaces of Reference Example 1, Examples 1 and 2, and Comparative Examples 1 and 2 are shown in Table 3. The polyrotaxane surface of Comparative Example 1 had the least negative charge, and Example 2 had the most negative charge of all surfaces. While the amino group should be positively charged at pH 7.4, the polyrotaxane surface of Comparative Example 1 was negatively charged. This is considered to be due to the negative charge of the underlying substrate (TCPS) of the coating and the negative charge of the unmodified PRX surface.

**[Table 3]**

| | Contact angle of water in atmosphere (θ) | Contact angle of water in water (θ) | Contact angle hysteresis (θ) | Zeta potential (mV) |
|---|---|---|---|---|
| Reference Example 1 | 93.8±1.3 | 75.5±1.5 | 18.3±1.1 | -24.6±2.8 |
| Comparative Example 1 | 80.8±2.1 | 43.0±1.4 | 37.8±2.8 | -9.9±0.7 |
| Comparative Example 2 | 85.0±1.7 | 60.0±2.4 | 25.0±3.9 | -29.3±2.8 |
| Example 1 | 83.5±1.1 | 52.9±4.0 | 30.7±5.1 | -16.3±6.3 |
| Example 2 | 86.5±2.7 | 22.2±2.0 | 64.3±3.8 | -33.6±3.4 |

### (3) Fibronectin Adsorption on Polyrotaxane Surface (Micro BCA Assay)

In order to examine fibronectin adsorption on the polyrotaxane surface, a PBS solution of human fibronectin (100 µg/mL) was incubated at 37°C for 3 hours on each of the surfaces of Reference Example 1, Examples 1 and 2, and Comparative Examples 1 and 2. Each well was washed three times with PBS to remove unadsorbed fibronectin. Adsorbed fibronectin was extracted by adding 5% SDS and a 0.1 NNaOH aqueous solution and incubating at 37°C for 1 hour according to the method described in J. Biomater. Sci. Polym. Ed., 2017, 28, 986-999 or ACS Biomater. Sci. Eng., 2018, 4, 1591-1597. The fibronectin concentrations were measured using a protein assay kit (Micro BCA (trade name), Thermo Scientific) with human fibronectin standards according to the manufacturer's instructions.

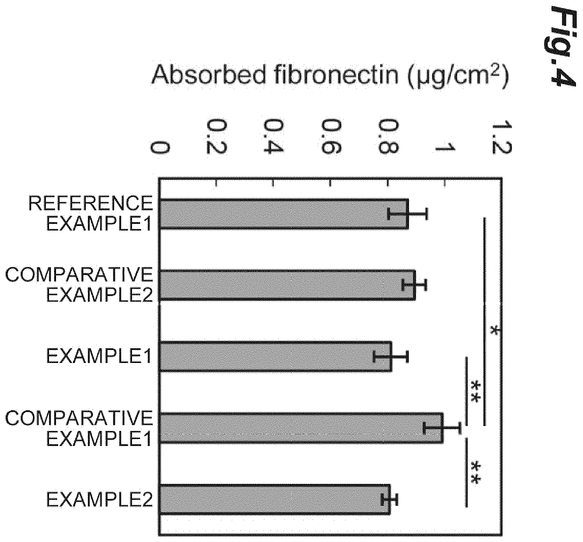

The results are shown in FIG. 4. The fibronectin was most adsorbed on the surface of Comparative Example 1. Fibronectin was negatively charged, and it was considered that a large amount of fibronectin was adsorbed by electrostatic interaction with a positively charged amino group. The fibronectin adsorption amounts on the polyrotaxane surfaces of Examples 1 and 2 and Comparative Example 2 were not significantly different from that of Reference Example 1.

### 2. Cell Response on Polyrotaxane Surface

### (1) Human Mesenchymal Stem Cell (hMSC) Adhesion and Proliferation on Polyrotaxane Surface

In order to evaluate initial adhesion and proliferation of hMSCs on each polyrotaxane surface, cells were seeded on each surface at a concentration of 6.0×10³ cells/cm² and cultured at 37°C for 3 days in a humidified atmosphere containing 5% CO₂ using a medium for hMSC proliferation BulletKit. The morphology of the cells was observed using a phase contrast microscope (BZ-X700, KEYENCE CORPORATION), the adhered cells were removed from the substrate by treatment with a trypsin/EDTA solution, and the number of hMSCs adhered to each surface was measured at intervals of one day using a hemocytometer.

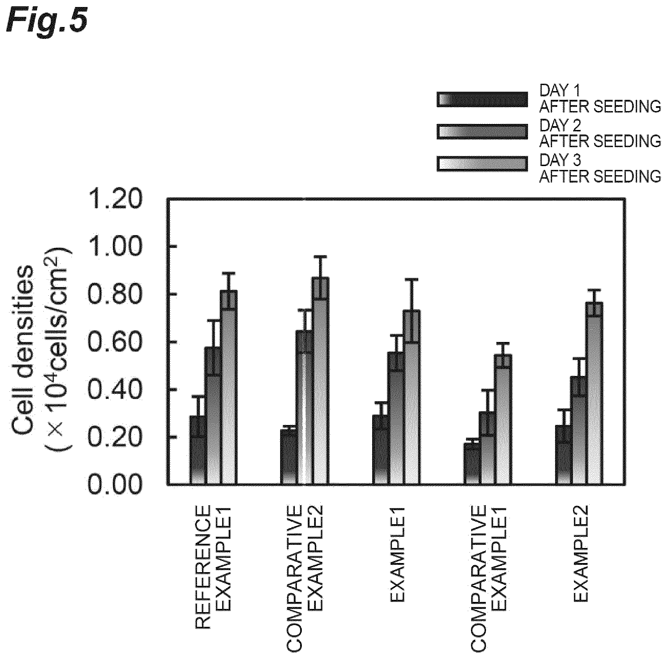

The results are shown in FIG. 5. On Day 1 after seeding, there was no significant difference in cell density between the polyrotaxane surfaces. On Day 3 of seeding, the cell density on the surface of Comparative Example 1 was the lowest.

### (2) Osteoblast Differentiation of hMSC on Polyrotaxane Surface

In order to induce osteoblast differentiation (osteogenic differentiation), hMSCs were seeded on each polyrotaxane surface at a density of 2.4×10⁴ cells/cm², and cultured for 5 days at 37°C in a humidified atmosphere containing 5% CO₂ using a medium for hMSC proliferation BulletKit until the density of cells became excessive confluent. The medium for proliferation was replaced with a medium for osteoblast differentiation, and the cells were incubated for 14 days. The medium was replaced with a fresh medium every 3 to 4 days. As a medium for osteoblast differentiation, Mesenchymal Stem Cell Osteogenic Differentiation Medium (C-28013) available from PromoCell GmbH (Heidelberg, Germany) was used.

After 14 days of differentiation induction, the cells were stained with Alizarin Red S to evaluate the extent of calcification of hMSC. The cells were washed twice with PBS and fixed by treatment with a 4% paraformaldehyde solution for 10 minutes at 23°C. The cells were washed twice with MilliQ water and stained with an Alizarin Red S solution for 10 minutes at 23°C. After removal of the stain solution, the cells were washed four times with MilliQ water and observed with a microscope. The stained area was calculated using ImageJ software. All measurement values were obtained on three different surfaces, and an average value of four different points on each surface was taken as an average value of each surface.

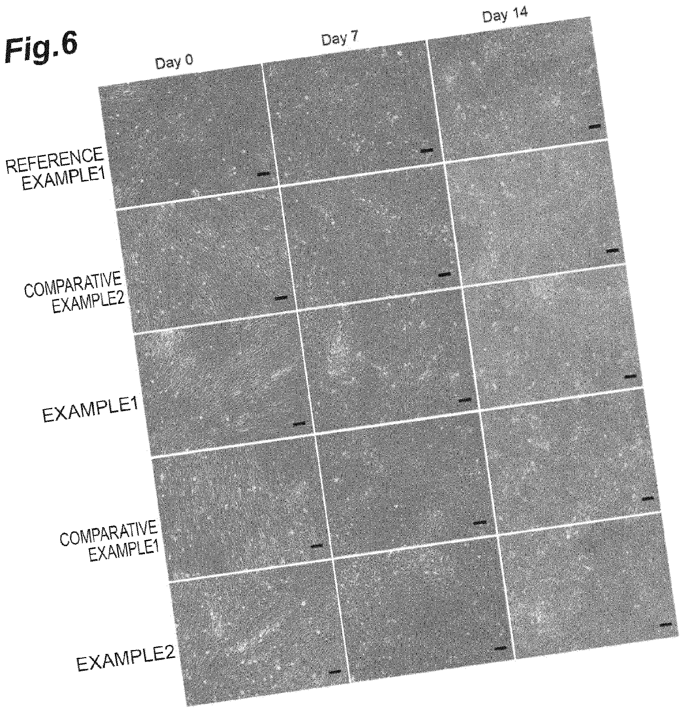

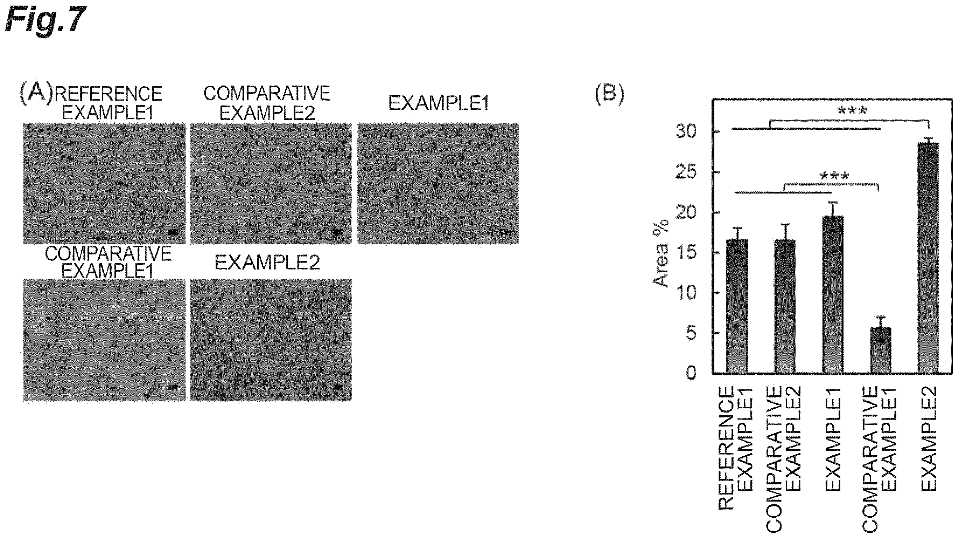

The results are shown in FIG. 6. After induction of osteogenic differentiation, the cell morphology changed from an elongated shape to a square shape and the cell density increased, particularly, on the surface of Example 2. In order to evaluate formation of bone nodules of hMSCs cultured on each surface, calcium nodules were stained using Alizarin Red S on Day 14 after osteoblast differentiation induction. FIG. 7(A) shows photographs stained with Alizarin Red S, and FIG. 7(B) is a graph comparing stained areas in respective polyrotaxane surfaces. Significant differences in stained area were observed, and the significant difference was the largest on the most negatively charged surface (Example 2), was the smallest on the most negatively uncharged surface (Comparative Example 1), and was moderate on other surfaces (Reference Example 1, Example 1, and Comparative Example 2).

### (3) Adipocyte Differentiation of hMSC on Polyrotaxane Surface

In order to induce adipocyte differentiation, hMSCs were seeded on each polyrotaxane surface at a density of 1.0×10⁴ cells/cm², and cultured for 5 days at 37°C in a humidified atmosphere containing 5% CO₂ using a medium for hMSC proliferation BulletKit. The medium for proliferation was replaced with a medium for adipocyte differentiation, and the cells were cultured for 15 days. The medium was replaced with a fresh medium every 3 to 4 days. As a medium for adipocyte differentiation, Mesenchymal Stem Cell Adipogenic Differentiation Medium 2 (C-28016) available from PromoCell GmbH (Heidelberg, Germany) was used.

After 15 days of differentiation induction, the cells were stained with Oil Red O to evaluate lipid droplet accumulation in cells. The cells were washed twice with PBS and fixed by treatment with a 4% paraformaldehyde solution for 10 minutes at 23°C. The cells were washed twice with PBS and washed once with 60% 2-propanol. The cells were stained with an Oil Red O solution for 20 minutes at 23°C and washed once with 60% 2-propanol. Finally, the cells were washed twice with PBS and observed in PBS using a microscope. The stained area was calculated using ImageJ software. All measurement values were obtained on three different surfaces, and an average value of three different points on each surface was taken as an average value of each surface.

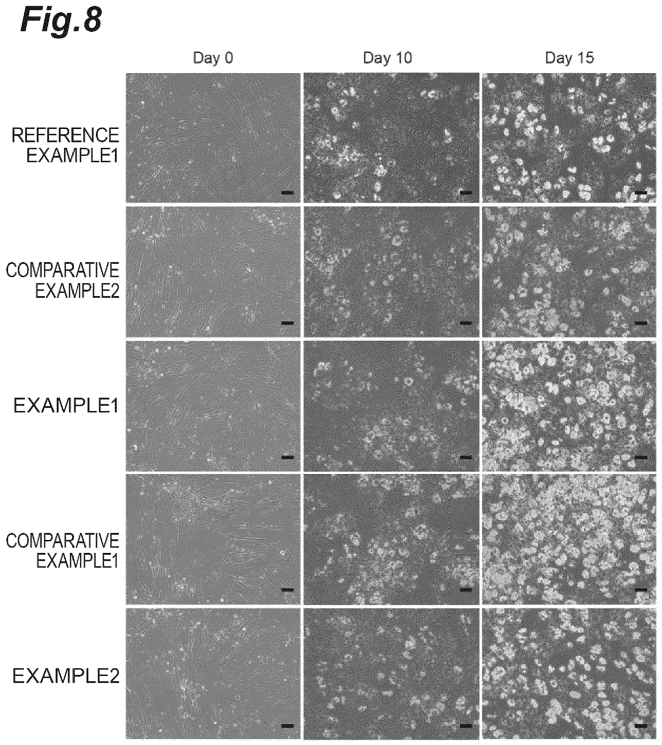

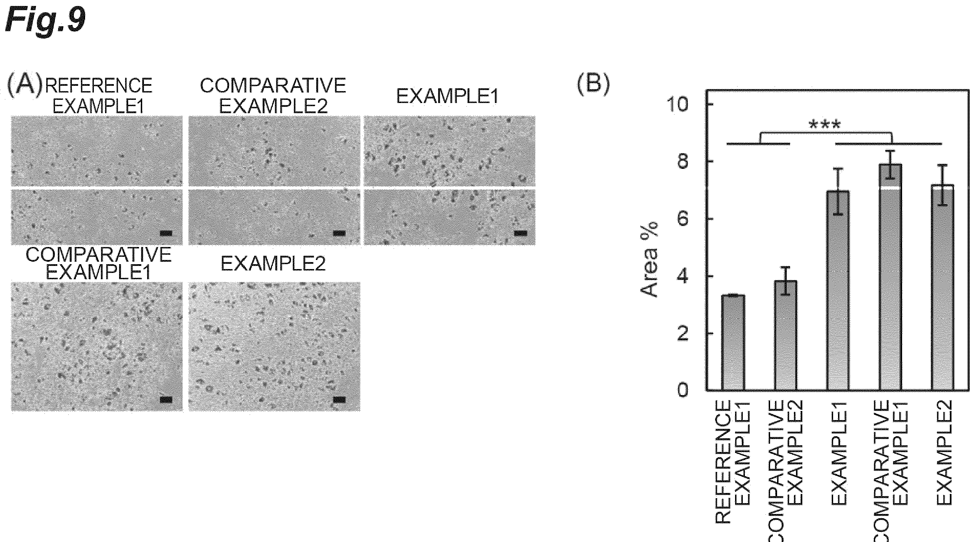

The results are shown in FIG. 8. Intracellular droplet formation associated with adipocyte differentiation was observed on all surfaces, and an increase in lipid droplets was observed according to the culture period. FIG. 9(A) shows photographs stained with Oil Red O, and FIG. 9(B) is a graph comparing stained areas in respective polyrotaxane surfaces. The stained areas on the surfaces of Comparative Example 1 and Examples 1 and 2 were larger than the stained areas on the surfaces of Reference Example 1 and Comparative Example 2. The stained area with Oil Red O tended to increase as the contact angle hysteresis increased. Since the difference in the contact angle hysteresis indicates a difference in molecular mobility of the surface, it is considered that the molecular mobility of, particularly, the polyrotaxane surfaces of Comparative Example 1 and Examples 1 and 2 is more suitable for promoting adipocyte differentiation.

## Claims

1. Use of a polyrotaxane represented by Formula (1) as a coating agent for promoting osteoblast differentiation or adipocyte differentiation of mesenchymal stem cells: 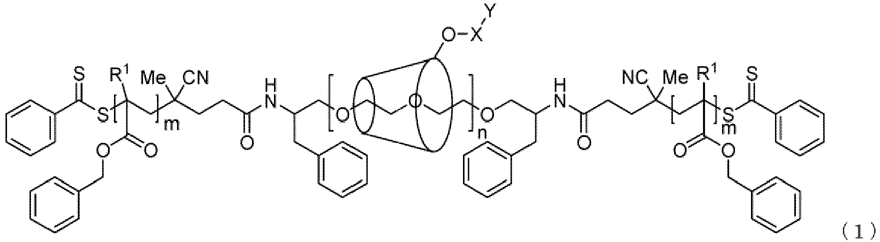
wherein R¹ is a hydrogen atom or a methyl group, m is 1 to 2000, and n is 10 to 500, 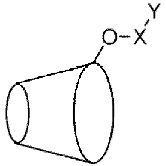 is a cyclodextrin in which at least one hydroxyl group is modified with a group represented by -X-Y, X is a divalent organic group, and Y is a hydroxyl group or a sulfo group,
wherein a number of cyclodextrins per polyrotaxane molecule is in the range of 5 to 150,
wherein promoting osteoblast differentiation includes culturing the mesenchymal stem cells on a substrate coated with a coating comprising the polyrotaxane represented by Formula (1) in a medium for osteoblast differentiation, and
wherein promoting adipocyte differentiation includes culturing the mesenchymal stem cells on a substrate coated with a coating comprising the polyrotaxane represented by Formula (1) in a medium for adipocyte differentiation.

2. The use according to claim 1, wherein the polyrotaxane comprises a cyclodextrin in which 1 to 18 hydroxyl groups are modified with the group represented by -X-Y.

3. The use according to claim 1 or 2, wherein the polyrotaxane has a number of threaded cyclodextrins of 5 to 100.

4. A cell culture method for promoting osteoblast differentiation or adipocyte differentiation of mesenchymal stem cells, the method comprising:
culturing mesenchymal stem cells on a surface of a substrate coated with a composition comprising a polyrotaxane represented by Formula (1): 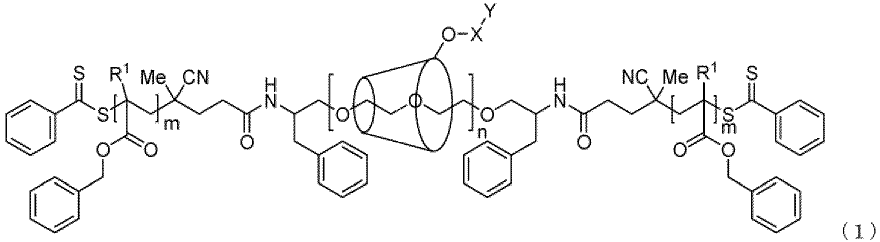
wherein R¹ is a hydrogen atom or a methyl group, m is 1 to 2000, and n is 10 to 500, 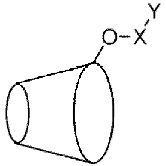
is a cyclodextrin in which at least one hydroxyl group is modified with a group represented by -X-Y, X is a divalent organic group, and Y is a hydroxyl group or a sulfo group;
wherein a number of cyclodextrins per polyrotaxane molecule is in the range of 5 to 150,
wherein for promoting osteoblast differentiation, the mesenchymal stem cells are cultured on the surface of the substrate in a medium for osteoblast differentiation, and
wherein for promoting adipocyte differentiation, the mesenchymal stem cells are cultured on the surface of the substrate in a medium for adipocyte differentiation.

5. The method according to claim 4, comprising coating the composition comprising a polyrotaxane represented by Formula (1) onto the surface of the substrate.

6. The method according to claim 4 or 5, wherein the polyrotaxane comprises a cyclodextrin in which 1 to 18 hydroxyl groups are modified with the group represented by -X-Y.

7. The method according to any one of claims 4 to 6, wherein the polyrotaxane has a number of threaded cyclodextrins of 5 to 120.

8. Use of a culture substrate in a cell culture method for promoting osteoblast differentiation or adipocyte differentiation of mesenchymal stem cells, the culture substrate comprising a substrate coated with a composition comprising a polyrotaxane represented by Formula (1): 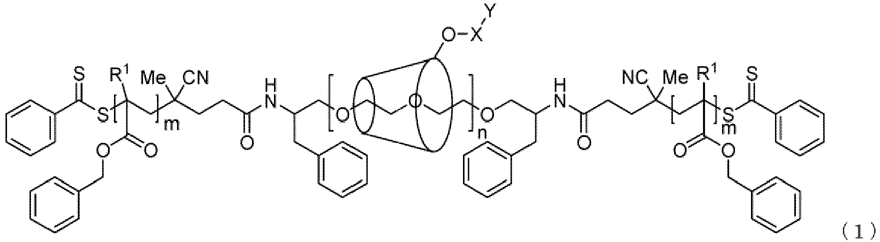
wherein R¹ is a hydrogen atom or a methyl group, m is 1 to 2000, and n is 10 to 500, 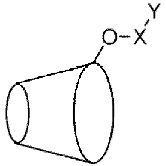 is a cyclodextrin in which at least one hydroxyl group is modified with a group represented by -X-Y, X is a divalent organic group, and Y is a hydroxyl group or a sulfo group,
wherein a number of cyclodextrins per polyrotaxane molecule is in the range of 5 to 150,
wherein promoting osteoblast differentiation includes culturing the mesenchymal stem cells on a substrate coated with a coating comprising the polyrotaxane represented by Formula (1) in a medium for osteoblast differentiation, and
wherein promoting adipocyte differentiation includes culturing the mesenchymal stem cells on a substrate coated with a coating comprising the polyrotaxane represented by Formula (1) in a medium for adipocyte differentiation.

9. The use according to claim 8, wherein the polyrotaxane comprises a cyclodextrin in which 1 to 18 hydroxyl groups are modified with the group represented by -X-Y.

10. The use according to claim 8 or 9, wherein the polyrotaxane has a number of threaded cyclodextrins of 5 to 120.

## Patentansprüche

1. Verwendung eines Polyrotaxans, das durch Formel (1) dargestellt ist, als Beschichtungsmittel zur Förderung der Osteoblastendifferenzierung oder Adipozytendifferenzierung mesenchymaler Stammzellen: 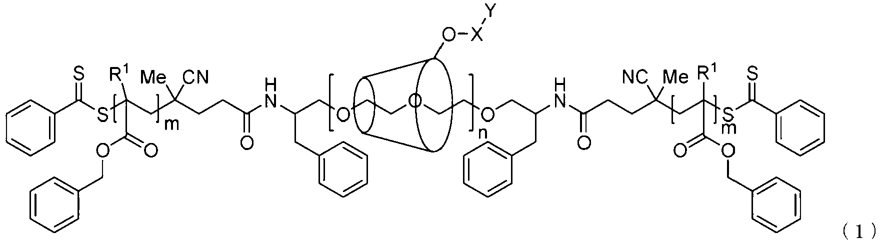
wobei R¹ ein Wasserstoffatom oder eine Methylgruppe ist, m 1 bis 2000 beträgt und n 10 bis 500 beträgt, 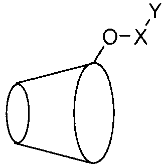 ein Cyclodextrin ist, bei dem mindestens eine Hydroxylgruppe mit einer durch - X-Y dargestellten Gruppe modifiziert ist, wobei X eine zweiwertige organische Gruppe ist und Y eine Hydroxylgruppe oder eine Sulfogruppe ist,
wobei eine Anzahl von Cyclodextrinen pro Polyrotaxanmolekül im Bereich von 5 bis 150 liegt,
wobei die Förderung der Osteoblastendifferenzierung Kultivieren der mesenchymalen Stammzellen auf einem Substrat, das mit einer Beschichtung beschichtet ist, die das durch Formel (1) dargestellte Polyrotaxan umfasst, in einem Medium für die Osteoblastendifferenzierung beinhaltet, und
wobei die Förderung der Adipozytendifferenzierung Kultivieren der mesenchymalen Stammzellen auf einem Substrat, das mit einer Beschichtung beschichtet ist, die das durch Formel (1) dargestellte Polyrotaxan umfasst, in einem Medium für die Adipozytendifferenzierung beinhaltet.

2. Verwendung nach Anspruch 1, wobei das Polyrotaxan ein Cyclodextrin umfasst, in dem 1 bis 18 Hydroxylgruppen mit der durch -X-Y dargestellten Gruppe modifiziert sind.

3. Verwendung nach Anspruch 1 oder 2, wobei das Polyrotaxan eine Anzahl von aneinandergereihten Cyclodextrinen von 5 bis 100 aufweist.

4. Zellkulturverfahren zur Förderung der Osteoblastendifferenzierung oder der Adipozytendifferenzierung von mesenchymalen Stammzellen, wobei das Verfahren Folgendes umfasst:
Kultivieren mesenchymaler Stammzellen auf einer Oberfläche eines Substrats, das mit einer Zusammensetzung beschichtet ist, die ein durch Formel (1) dargestelltes Polyrotaxan umfasst: 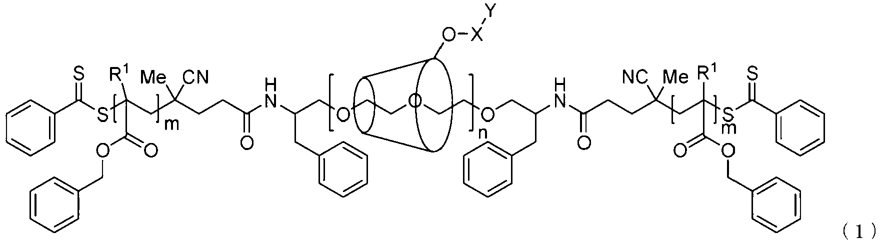
wobei R¹ ein Wasserstoffatom oder eine Methylgruppe ist, m 1 bis 2000 beträgt und n 10 bis 500 beträgt, 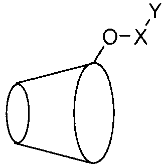
ein Cyclodextrin ist, bei dem mindestens eine Hydroxylgruppe mit einer durch -X-Y dargestellten Gruppe modifiziert ist, wobei X eine zweiwertige organische Gruppe ist und Y eine Hydroxylgruppe oder eine Sulfogruppe ist;
wobei eine Anzahl von Cyclodextrinen pro Polyrotaxanmolekül im Bereich von 5 bis 150 liegt,
wobei zur Förderung der Osteoblastendifferenzierung die mesenchymalen Stammzellen auf der Oberfläche des Substrats in einem Medium für die Osteoblastendifferenzierung kultiviert werden, und
wobei zur Förderung der Adipozytendifferenzierung die mesenchymalen Stammzellen auf der Oberfläche des Substrats in einem Medium für die Adipozytendifferenzierung kultiviert werden.

5. Verfahren nach Anspruch 4, umfassend Auftragen der Zusammensetzung, die ein durch Formel (1) dargestelltes Polyrotaxan umfasst, auf die Oberfläche eines Substrats.

6. Verfahren nach Anspruch 4 oder 5, wobei das Polyrotaxan ein Cyclodextrin umfasst, in dem 1 bis 18 Hydroxylgruppen mit der durch -X-Y dargestellten Gruppe modifiziert sind.

7. Verfahren nach einem der Ansprüche 4 bis 6, wobei das Polyrotaxan eine Anzahl von aneinandergereihten Cyclodextrinen von 5 bis 120 aufweist.

8. Verwendung eines Kultursubstrats in einem Zellkulturverfahren zur Förderung der Osteoblastendifferenzierung oder Adipozytendifferenzierung von mesenchymalen Stammzellen, wobei das Kultursubstrat ein Substrat umfasst, das mit einer Zusammensetzung beschichtet ist, die ein durch Formel (1) dargestelltes Polyrotaxan umfasst: 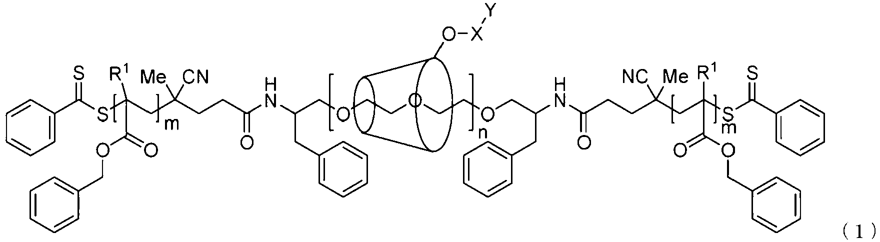
wobei R¹ ein Wasserstoffatom oder eine Methylgruppe ist, m 1 bis 2000 beträgt und n 10 bis 500 beträgt, 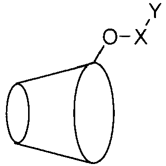 ein Cyclodextrin ist, bei dem mindestens eine Hydroxylgruppe mit einer durch - X-Y dargestellten Gruppe modifiziert ist, wobei X eine zweiwertige organische Gruppe ist und Y eine Hydroxylgruppe oder eine Sulfogruppe ist,
wobei eine Anzahl von Cyclodextrinen pro Polyrotaxanmolekül im Bereich von 5 bis 150 liegt,
wobei die Förderung der Osteoblastendifferenzierung Kultivieren der mesenchymalen Stammzellen auf einem Substrat, das mit einer Beschichtung beschichtet ist, die das durch Formel (1) dargestellte Polyrotaxan umfasst, in einem Medium für die Osteoblastendifferenzierung beinhaltet, und
wobei die Förderung der Adipozytendifferenzierung Kultivieren der mesenchymalen Stammzellen auf einem Substrat, das mit einer Beschichtung beschichtet ist, die das durch Formel (1) dargestellte Polyrotaxan umfasst, in einem Medium für die Adipozytendifferenzierung beinhaltet.

9. Verwendung nach Anspruch 8, wobei das Polyrotaxan ein Cyclodextrin umfasst, in dem 1 bis 18 Hydroxylgruppen mit der durch -X-Y dargestellten Gruppe modifiziert sind.

10. Verwendung nach Anspruch 8 oder 9, wobei das Polyrotaxan eine Anzahl von aneinandergereihten Cyclodextrinen von 5 bis 120 aufweist.

## Revendications

1. Utilisation d'un polyrotaxane représenté par la formule (1) comme agent de revêtement pour favoriser la différenciation ostéoblastique ou la différenciation adipocytaire de cellules souches mésenchymateuses : 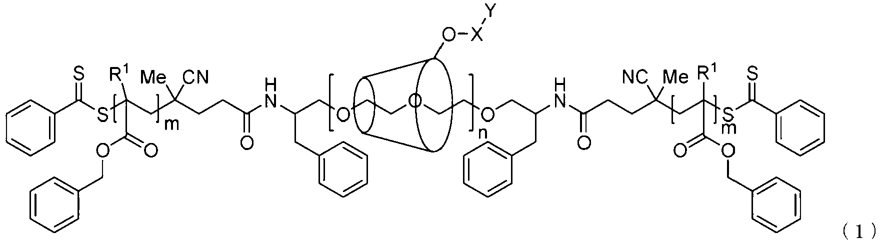
dans laquelle R¹ est un atome d'hydrogène ou un groupe méthyle, m est compris entre 1 et 2000, et n est compris entre 10 et 500, 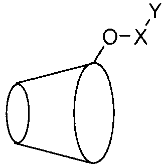 est une cyclodextrine dans laquelle au moins un groupe hydroxyle est modifié par un groupe représenté par -X-Y, X est un groupe organique divalent, et Y est un groupe hydroxyle ou un groupe sulfo,
dans laquelle le nombre de cyclodextrines par molécule de polyrotaxane est compris entre 5 et 150,
dans laquelle la promotion de la différenciation ostéoblastique comprend la culture des cellules souches mésenchymateuses sur un substrat recouvert d'un revêtement comprenant le polyrotaxane représenté par la formule (1) dans un milieu pour la différenciation ostéoblastique, et
dans laquelle la promotion de la différenciation adipocytaire comprend la culture des cellules souches mésenchymateuses sur un substrat revêtu d'un revêtement comprenant le polyrotaxane représenté par la formule (1) dans un milieu pour la différenciation adipocytaire.

2. Utilisation selon la revendication 8, dans laquelle le polyrotaxane comprend une cyclodextrine dans laquelle 1 à 18 groupes hydroxyles sont modifiés par le groupe représenté par -X-Y.

3. Utilisation selon la revendication 1 ou 2, dans laquelle le polyrotaxane a un nombre de cyclodextrines enfilées compris entre 5 à 100.

4. Procédé de culture cellulaire pour favoriser la différenciation ostéoblastique ou la différenciation adipocytaire de cellules souches mésenchymateuses, le procédé comprenant :
la culture de cellules souches mésenchymateuses sur une surface d'un substrat revêtu d'une composition comprenant un polyrotaxane représenté par la formule (1) : 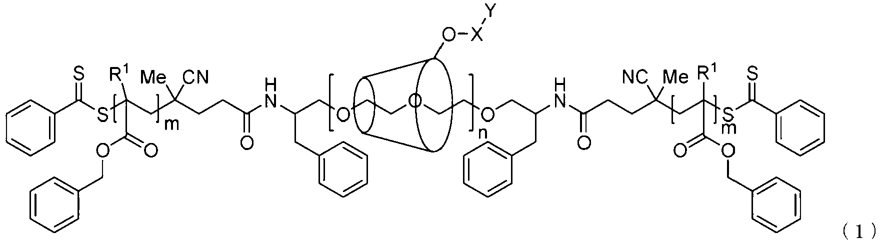
dans lequel R¹ est un atome d'hydrogène ou un groupe méthyle, m est compris entre 1 et 2000, et n est compris entre 10 et 500, 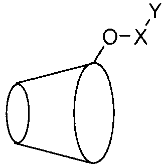
est une cyclodextrine dans laquelle au moins un groupe hydroxyle est modifié par un groupe représenté par -X-Y, X est un groupe organique divalent, et Y est un groupe hydroxyle ou un groupe sulfo ;
dans lequel le nombre de cyclodextrines par molécule de polyrotaxane est compris entre 5 et 150,
dans lequel, pour favoriser la différenciation ostéoblastique, les cellules souches mésenchymateuses sont cultivées à la surface du substrat dans un milieu pour la différenciation ostéoblastique, et
dans lequel, pour favoriser la différenciation adipocytaire, les cellules souches mésenchymateuses sont cultivées à la surface du substrat dans un milieu pour la différenciation adipocytaire.

5. Procédé selon la revendication 4, comprenant le revêtement de la composition comprenant un polyrotaxane représenté par la formule (1) sur la surface du substrat.

6. Procédé selon la revendication 4 ou 5, dans lequel le polyrotaxane comprend une cyclodextrine dans laquelle 1 à 18 groupes hydroxyles sont modifiés par le groupe représenté par -X-Y.

7. Procédé selon l'une quelconque des revendications 4 à 6, dans lequel le polyrotaxane a un nombre de cyclodextrines enfilées compris entre 5 à 120.

8. Utilisation d'un substrat de culture dans un procédé de culture cellulaire pour favoriser la différenciation ostéoblastique ou la différenciation adipocytaire des cellules souches mésenchymateuses, le substrat de culture comprenant un substrat revêtu d'une composition comprenant un polyrotaxane représenté par la formule (1) : 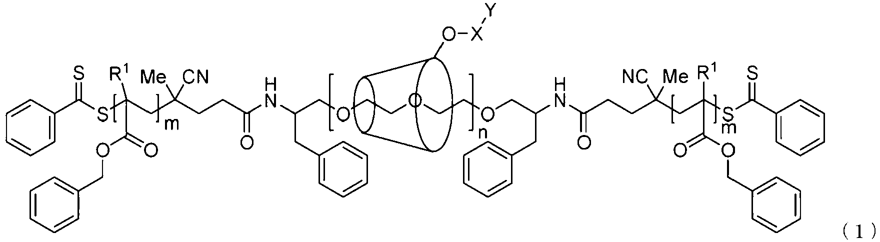
dans laquelle R¹ est un atome d'hydrogène ou un groupe méthyle, m est compris entre 1 et 2000, et n est compris entre 10 et 500, 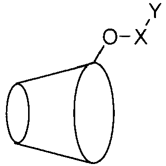 est une cyclodextrine dans laquelle au moins un groupe hydroxyle est modifié par un groupe représenté par -X-Y, X est un groupe organique divalent, et Y est un groupe hydroxyle ou un groupe sulfo,
dans laquelle un nombre de cyclodextrines par molécule de polyrotaxane est compris entre 5 et 150,
dans laquelle la promotion de la différenciation ostéoblastique comprend la culture des cellules souches mésenchymateuses sur un substrat recouvert d'un revêtement comprenant le polyrotaxane représenté par la formule (1) dans un milieu pour la différenciation ostéoblastique, et
dans laquelle la promotion de la différenciation adipocytaire comprend la culture des cellules souches mésenchymateuses sur un substrat revêtu d'un revêtement comprenant le polyrotaxane représenté par la formule (1) dans un milieu pour la différenciation adipocytaire.

9. Utilisation selon la revendication 8, dans laquelle le polyrotaxane comprend une cyclodextrine dans laquelle 1 à 18 groupes hydroxyles sont modifiés par le groupe représenté par -X-Y.

10. Utilisation selon la revendication 8 ou 9, dans laquelle le polyrotaxane a un nombre de cyclodextrines enfilées compris entre 5 à 120.
